(19) Europäisches Patentamt
European Patent Office
Office européen des brevets

(11) Veröffentlichungsnummer: **0 208 263**
**B1**

# (12) EUROPÄISCHE PATENTSCHRIFT

(45) Veröffentlichungstag der Patentschrift: **06.09.89**

(51) Int. Cl.[4]: **C 07 D 498/10**, C 07 D 519/00 // (C07D498/10, 263:00, 221:00)

(21) Anmeldenummer: **86109072.8**

(22) Anmeldetag: **03.07.86**

(54) **Verfahren zur Herstellung von 1-Oxa-3,8-diaza-4-oxo-spiro-[4,5]decan-Verbindungen.**

(30) Priorität: **10.07.85 DE 3524543**

(43) Veröffentlichungstag der Anmeldung: **14.01.87 Patentblatt 87/3**

(45) Bekanntmachung des Hinweises auf die Patenterteilung: **06.09.89 Patentblatt 89/36**

(84) Benannte Vertragsstaaten: **BE CH DE FR GB IT LI NL**

(56) Entgegenhaltungen:
**DE-A- 2 933 732**
**DE-A- 3 149 453**

**Phase transfer catalyses (2. Auflage) Dehmlov and Dehmlov, Verlag Chemie, Weinheim, Deerfield Beach, Florida, Basel.**
**Phase transfer catayses, Starks and Liotta, Academic Press, New York 1978**
**Principals and applications of homogeneon catalyses, Nakomma and Tsutsui, John Wiley 1980**
**Römpps Chemie Lexikon, Band 4, Seite 3122**

(73) Patentinhaber: **HOECHST AKTIENGESELLSCHAFT, Postfach 80 03 20, D-6230 Frankfurt am Main 80 (DE)**

(72) Erfinder: **Ertl, Josef, Dr., Eichenstrasse 14, D-8857 Wertingen (DE)**
Erfinder: **Wiezer, Hartmut, Dr., Am Herzhain 1b, D-6239 Eppstein/Taunus (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

**Beschreibung**

Die Erfindung betrifft ein Verfahren zur Herstellung von 1-Oxa-3,8-diaza-4-oxo-spiro[4,5]decan-Verbindungen, die als Lichtschutzmittel für Polymere oder als Zwischenprodukte für die Herstellung von Kunststoffadditiven verwendet werden können.

Verbindungen der Formel

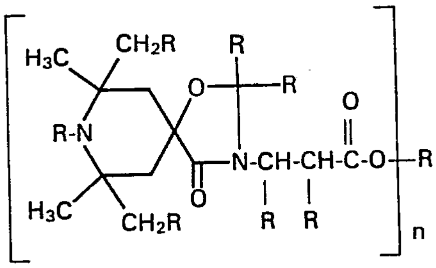

sind bekannt (vgl. Deutsche Offenlegungsschrift 3 149 453). Allerdings ist das Verfahren zu ihrer Herstellung kompliziert, da während der Reaktion das Reaktionsmedium mehrfach gewechselt wird, was zusätzliche Extraktionen und Destillationen bedingt.

Es wurde nun gefunden, daß die Herstellungsreaktion schneller und vollständiger verläuft, wenn man als Lösemittel einen bei Raumtemperatur flüssigen aromatischen Kohlenwasserstoff verwendet und einen Phasentransferkatalysator zusetzt.

Gegenstand der Erfindung ist somit ein Verfahren zur Herstellung von 1-Oxa-3,8-diaza-4-oxo-spiro[4,5]-decan-Verbindungen der Formel I

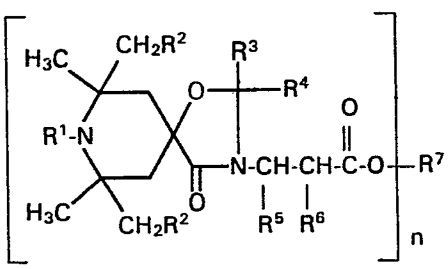 (I)

worin

n eine ganze Zahl von 1 bis 4 ist,

$R^1$ Wasserstoff, $C_1$-$C_4$-Alkyl, Benzyl, Allyl, $C_2$-$C_{30}$-Alkanoyl, $C_3$-$C_{20}$-Alkenoyl, $C_7$-$C_{11}$-Aroyl, $C_8$-$C_{14}$-Arylalkanoyl oder $C_8$-$C_{20}$-Alkylaryl ist.

$R^2$ Wasserstoff oder $C_1$-$C_4$-Alkyl bedeutet,

$R^3$ Wasserstoff, $C_1$-$C_{18}$-Alkyl, $C_5$-$C_{12}$-Cycloalkyl, eine Phenyl- oder Naphthylgruppe, die durch Chlor oder $C_1$-$C_4$-Alkyl substituiert sein kann, oder eine gegebenenfalls durch $C_1$-$C_4$-Alkyl substituierte $C_7$-$C_{12}$-Phenylalkylengruppe ist,

$R_4$ Wasserstoff, $C_1$-$C_4$-Alkyl, $C_5$-$C_{12}$-Cycloalkyl, durch -COOH, Carb-$C_1$-$C_4$-alkoxi oder Carbamoyl substituiertes $C_1$-$C_3$-Alkenyl, eine Phenyl-, Naphthyl- oder Pyridylgruppe, die durch $C_1$-$C_4$-Alkoxi oder $C_1$-$C_4$-Alkyl substituiert sein kann, oder eine $C_7$-$C_{12}$-Phenylalkylgruppe, die durch $C_1$-$C_4$-Alkyl substituiert sein kann, bedeutet, oder

$R^3$ und $R^4$ zusammen mit dem sie bindenden Kohlenstoffatom eine Cycloalkylgruppe, die durch eine bis vier $C_1$-$C_4$-Alkylgruppen substituiert sein kann, oder einen Rest der Formel II

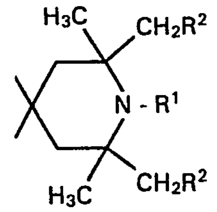 (II),

worin $R^1$ und $R^2$ die obengenannte Bedeutung haben, bilden

$R^5$ Wasserstoff, Methyl, Phenyl oder Carb-$C_1$-$C_{21}$-Alkoxi ist,

$R^6$ Wasserstoff oder Methyl bedeutet,

$R^7$ bei n = 1

Wasserstoff, $C_1$-$C_{21}$-Alkyl, $C_2$-$C_{22}$-Alkenyl, $C_7$-$C_{18}$-Phenylalkyl, $C_5$-$C_{12}$-Cycloalkyl, Phenyl, Naphthyl, $C_7$-$C_{18}$-Alkylphenyl, ein Rest der Formel

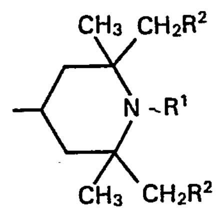

worin $R^1$ und $R^2$ die obige Bedeutung haben, $C_2$-$C_{20}$-Alkyl, welches unterbrochen ist durch -O- oder -N-
|
$R^8$

und/oder substituiert durch einen Rest der Formel III

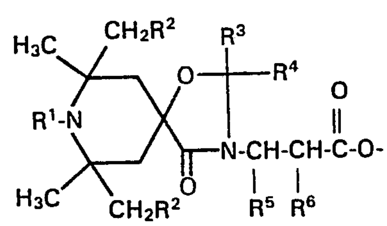 (III)

oder durch $C_1$-$C_{21}$-Alkylcarbonyl, wobei $R^1$, $R^2$, $R^3$, $R^4$, $R^5$ und $R^6$ die obige Bedeutung haben und $R^8$ Wasserstoff oder $C_1$-$C_{10}$-Alkyl ist, bedeutet,

$R^7$ bei n = 2

geradkettiges oder verzweigtes $C_1$-$C_{30}$-Alkylen, $C_2$-$C_{30}$-Alkenylen, Phenyldialkylen, wobei diese Reste durch -O- oder -N-, worin $R^8$ die obige Bedeutung
|
$R^8$
hat, unterbrochen sein können, bedeutet,

$R^7$ bei n = 3 oder 4

einen Rest der Formeln IV, V, VI oder VII

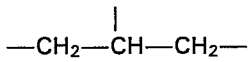 (IV),

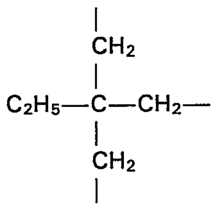 (V),

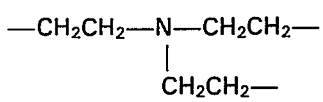 (VI),

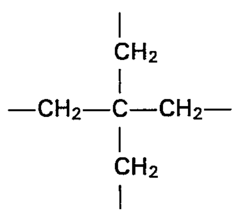 (VII),

bedeutet,

durch Umsetzung von Verbindungen der Formel VIII

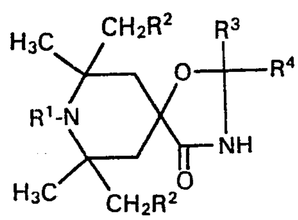 (VIII),

mit Verbindungen der Formel IX

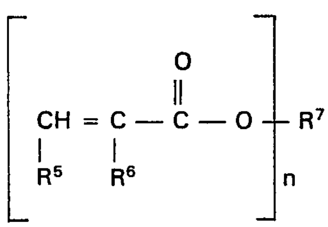 (IX),

wobei n, $R^1$, $R^2$, $R^3$, $R^4$, $R^5$, $R^6$ und $R^7$ die obengenannte Bedeutung haben, in einer Menge von 1/n bis 10/n, wobei n die obengenannte Bedeutung hat, bezogen auf 1 Mol des Verbindung der Formel VIII, in einem inerten Lösemittel bei einer Temperatur von 30 bis 150°C in Gegenwart von 1 bis 30 Mol-%, bezogen auf eine Verbindung der Formel VIII, eines basischen Katalysators, das dadurch gekennzeichnet ist, daß die Umsetzung in Gegenwart von 0,05 bis 20 Mol-%, bezogen auf die Verbindung VIII, eines Phasentransferkatalysators in einem bei Raumtemperatur flüssigen aromatischen Kohlenwasserstoff durchgeführt wird.

$R^1$ ist bevorzugt Wasserstoff, $C_1$-$C_4$-Alkyl, $C_2$-$C_{18}$-Alkanoyl, beispielsweise Methyl, Ethyl, Propyl, Butyl, Acetyl, Propionyl, Butyryl, Lauroyl, Stearoyl, besonders bevorzugt Wasserstoff oder einer der genannten Säurereste. Insbesondere ist $R^1$ Wasserstoff.

$R^2$ ist bevorzugt Wasserstoff oder $C_1$-$C_4$-Alkyl, beispielsweise Methyl, Ethyl, Propyl, Butyl. Insbesondere ist $R^2$ Wasserstoff.

$R^3$ und $R^4$ sind unabhängig voneinander $C_1$-$C_{18}$-Alkyl, $C_5$-$C_{12}$-Cycloalkyl oder Phenyl, beispielsweise Ethyl, Butyl, Octyl, Lauryl, Stearyl, Cyclohexyl, Cyclodecyl, besonders bevorzugt $C_1$-$C_7$-Alkyl. Insbesondere sind $R^3$ und $R^4$ $C_1$-$C_4$-Alkyl, beispielsweise Methyl.

$R^3$ und $R^4$ zusammen mit dem sie bindenden Kohlenstoffatom sind bevorzugt $C_5$-$C_{12}$-Cycloalkylen, besonders bevorzugt $C_6$- bzw. $C_{12}$-Cycloalkylen, insbesondere Cyclododecylen.

$R^5$ ist bevorzugt Wasserstoff, Methyl oder Phenyl, besonders bevorzugt Wasserstoff.

$R^6$ ist bevorzugt Wasserstoff oder Methyl. Insbesondere ist $R^6$ Wasserstoff.

$R^7$ ist bevorzugt $C_1$-$C_{21}$-Alkyl, geradkettiges oder verzweigtes $C_1$-$C_{30}$-Alkylen, beispielsweise Methyl, Butyl, Octyl, Lauryl, Stearyl. Ethylen, Butylen, Hexylen, besonders bevorzugt $C_1$-$C_{15}$-Alkyl. Insbesondere ist $R^7$ $C_{12}$-$C_{14}$-Alkyl, beispielsweise Lauryl.

Geeignete Verbindungen der Formel VIII sind beispielsweise

2-Butyl-7,7,9,9-tetramethyl-1-oxa-3,8-diaza-4-oxo--spiro-]4,5]-decan
2-iso-Butyl-7,7,9,9-tetramethyl-1-oxa-3,8-diaza-4-oxo-spiro-[4,5]-decan
2-Pentyl-7, 7,9,9-tetramethy-1-oxa-3,8-diaza-4-oxo-spiro-[4,5]-decan
2-iso-Pentyl-7,7,9,9-tetramethyl-1-oxa-3,8-diaza-4-oxo-spiro-[4,6]-decan
2-Hexyl-7,7,9,9-tetramethyl-1-oxa-3,8-diaza-4-oxo-spiro-[4,5]-decan
2-Heptyl-7,7,9,9-tetramethyl-1-oxa-3,8-diaza-4-oxo-spiro-[4,6]-decan
2-iso-Heptyl-7,7,9,9-tetramethyl-1-oxa-3,8-diaza-4-oxo-spiro-[4,5]-decan
2-Nonyl-7,7,9,9-tetramethyl-1-oxa-3,8-diaza-4-oxo-spiro-[4,5]-decan
2-iso-Nonyl-7,7,9,9-tetramethyl-1-oxa-3,8-diaza-4-oxo-spiro-[4,5]-decan
2-Undecyl-7,7,9,9-tetramethyl-1-oxa-3,8-diaza-4-oxo-spiro-[4,5]-decan
2-Phenyl-7,7,9,9-tetramethyl-1-oxa-3,8-diaza-4-oxo-spiro-[4,5]-decan
2-(4-Chlor-phenyl)-7,7,9,9-tetramethyl-1-oxa-3,8-diaza-4-oxo-spiro-[4,5]-decan
2-Ethyl-2,7,7,9,9-pentamethyl-1-oxca-3,8-diaza-4-oxo-spiro-[4,6]-decan
2-Propyl-2,7,7,9,9-pentamethyl-1-oxa-3,8-diaza-4-oxo-spiro-[4,5]-decan
2-iso-Propyl-2,7,7,9,9-pentamethyl-1-oxa-3,8-diaza-4-oxo-spiro-[4,5]-decan
2-Butyl-2,7,7,9,9-pentamethyl-1-oxa-3,8-diaza-4-oxo-spiro-[4,6]-decan
2-iso-Butyl-2,7,7,9,9-pentamethyl-1-oxa-3,8-diaza-4-oxo-spiro-[4,5]-decan
2-Pentyl-2,7,7,9,9-pentamethyl-1-oxa-3,8-diaza-4-oxo-spiro-[4,5]-decan

2-Hexyl-2,7,7,9,9-pentamethyl-1-oxa-3,8-diaza-4-oxo-spiro-[4,5]-decan
2-Nonyl-2,7,7,9,9-pentamethyl-1-oxa-3,8-diaza-4-oxo-spiro-[4,5]-decan
2,2,7,7,9,9-Hexamethyl-1-oxa-3,8-diaza-4-oxo-spiro-[4,5]-decan
2,2,7,7,9,9-Heptamethyl-1-oxa-3,8-diaza-4-oxo-spiro-[4,5]-decan
2,2-Diethyl-7,7,9,9-tetramethyl-1-oxa-3,8-diaza-4-oxo-spiro-[4,5]-decan
2,2-Dipropyl-7,7,9,9-tetramethyl-1-oxa-3,8-diaza-4-oxo-spiro-[4,6]-decan
2,2-Dibutyl-7,7,9,9-tetramethyl-1-oxa-3,8-diaza-4-oxo-spiro-[4,5]-decan
2-Ethyl-2-pentyl-7,7,9,9-tetramethyl-1-oxa-3,8-diaza-4-oxo-spiro-[4,5]-decan
2,2-Dibenzyl-7,7,9,9-tetramethyl-1-oxa-3,8-diaza-4-oxo-spiro-[4,5]-decan
2,2,4,4-Tetramethyl-7-oxa-3,13-diaza-14-oxo-dispiro-[5,1,4,2]-tetradecan
2,2,4,4-Tetramethyl-7-oxa-3,14-diaza-15-oxo-dispiro-[5,1,5,2]-pentadecan
2,2,4,4-Tetramethyl-7-oxa-3,20-diaza-21-oxo-dispiro-[5,1,11,2]-heneicosan
2,2,7,7,9,9-Hexamethyl-1-oxa-3,8-diaza-4-oxo-8-acetyl-spiro-[4,5]-decan
2,2,4,4-Tetramethyl-7-oxa-3,14-diaza-15-oxo-3-acetyl-di-spiro-[5,1,5,2] -pentadecan
2,2,4,4-Tetramethyl-7-oxa-3,20-diaza-21 oxo-3-acetyl-di-spiro-[5,1,11,2]-heneicosan .

Geeignete Verbindungen der Formel IX sind beispielsweise
Acrylsäuremethylester
Acrylsäurethylester
Acrylsäure-n-butylester
Acrylsäure-iso-butylester
Acrylsäure-tert.-butylester
Acrylsäure-2-ethylhexylester
Acrylsäureoctylester
Acrylsäurelaurylester
Acrylsäuremyristylester
Acrylsäure-2-diethylaminoethylester
Methacrylsäuremethylester
Methacrylsäureethylester
Methacrylsäure-n-butylester
Methacrylsäure-iso-butylester
Methacrylsäure-tert.-butylester
Methacrylsäurelaurylester
Methacrylsäure-cyclohexylester
Methacrylsäureallylester
Methacrylsäure-2-ethoxyethylester
Methacrylsäure-2-dimethykaminoethylester
Crotonsäuremethylester
Crotonsäureethylester
1,4-Butandioldiacrylat
1,6-Hexandioldiacrylat
2-Ethyl-2-hydroxymethyl-1,3-propandiol-triacrylat
Diethylenglykoldiacrylat
Pentaerythrittriacrylat
Pentaerythrittetraacrylat
Ethylenglykoldimethacrylat
1,4-Butandioldimethacrylat
1,6-Hexandioldimethacrylat
Diethylenglykoldimethacrylat
Triethylenglykoldimethacrylat
Tripropylenglykoldiacrylat
Trimethylolpropantrimethacrylat
Acrylsäure-2,2,6,6-tetramethylpiperid-4-yl-ester
Crotonsäure-2,2,6,6-tetramethylpiperid-4-yl-ester
Methacrylsäure-2,2,6,6-tetramethylpiperid-4-yl-ester.

Für das erfindungsgemäße Verfahren wird als Lösemittel ein bei Raumtemperatur flüssiger aromatischer Kohlenwasserstoff verwendet, vorzugsweise Toluol oder Xylol.

Als Phasentransferkatalysator wird vorzugsweise zugesetzt ein Polyethylenglykoldialkylether, ein substituiertes Phosphoniumsalz, beispielsweise Tetraalkylphosphoniumhalogenid oder ein substituiertes Ammoniumsalz, beispielsweise Tetraalkylammoniumhalogenid oder Trialkylbenzylammoniumhalogenid. Insbesondere wird Triethylbenzylammoniumchlorid oder ein Tetraalkylphosphoniumbromid zugesetzt. Die Menge beträgt 0,05 bis 20, vorzugsweise 0,1 bis 10, insbesondere 1 bis 10 Mol-%, bezogen auf die Verbindung der Formel VIII.

Die Verbindung IX wird in einer Menge von 1/n bis 10/n, vorzugsweise 1/n bis 3/n, insbesondere 1/n bis 1,5/n Mol, bezogen auf 1 Mol der Verbindung VIII, eingesetzt. n hat die oben angegebene Bedeutung.

Die Reaktionstemperatur beträgt 30 bis 150, vorzugsweise 50 bis 120, insbesondere 70 bis 120°C.

Die Reaktion wird in Gegenwart eines basischen Katalysators durchgeführt. Als solcher dient ein Alkalimetall, vorzugsweise Natrium, welches in einer Menge von 1 bis 30 Mol-%, vorzugsweise 2 bis 10 Mol-%, bezogen auf die Verbindung VIII, verwendet wird.

Das erfindungsgemäße Verfahren bringt gegenüber dem Stand der Technik erhebliche Vorteile. Zunächst wird nur ein einziges, technisch einfach zu handhabendes Lösemittel bzw. Lösemittelgemisch verwendet. Überraschenderweise wird durch den Phasentransferkatalysator bewirkt, daß die Reaktion wesentlich schneller und vor allem vollständiger abläuft, so daß die Verbindung IX nicht mehr im vielfachen Überschuß eingesetzt werden muß, sondern ein geringer Überschuß genügt. Trotzdem wird eine höhere Ausbeute erhalten und die Menge der Nebenprodukte verringert.

Die erfindungsgemäß hergestellten Verbindungen der Formel (I) werden vor allem als Lichtstabilisatoren eingesetzt, beispielsweise für Polyolefine, insbesondere Polyethylen und Polypropylen, Ethylen/Propylencopolymere, Polybutylen, sowie Polystyrol, chloriertes Polyethylen, sowie Polyvinylchlorid, Polyester, Polycarbonat, Polymethylmethacrylate, Polyphenylenoxide, Polyamide, Polyurethane, Polypropylenoxid, Polyacetale, Phenol-Formaldehydharze, Epoxidharze, Polyacrylnitril und entsprechende Copolymerisate sowie ABS-Terpolymere. Vorzugsweise verwendet man die erfindungsgemäß hergestellten Verbindungen zum Stabilisieren von Polypropylen, niedermolekularen und hochmolekularen Polyethylen, Ethylen-Propylen-Copolymeren, Polyvinylchlorid, Polyester, Polyamid, Polyurethanen, Polyacrylnitril, ABS, Terpolymeren von Acrylester, Styrol

und Acrylnitril, Copolymeren von Styrol und Acrylnitril oder Styrol und Butadien, insbesondere für Polypropylen, Polyethylen, Polyethylen, Ethylen-Propylencopolymer oder ABS.

Die erfindungsgemäß hergestellten Verbindungen können auch für die Stabilisierung von Naturstoffen, beispielsweise Kautschuk, verwendet werden, sowie auch für Schmieröle. Weiterhin eignen sie sich auch für die Stabilisierung von Lacken.

Als Lacke kommen alle in der Industrielackierung verwendeten Arten, vorzugsweise Einbrennlacke, infrage, wie sie in der Deutschen Offenlegungsschrift 3 149 453 aufgezählt sind.

Das Einbringen der erfindungsgemäß hergestellten Verbindungen in die zu schützenden Materialien erfolgt nach an sich bekannten Methoden, wobei auch Monomere oder Vorpolymerisate bzw. Vorkondensate mit diesen Stabilisatoren versehen werden können.

Neben den Verbindungen der Formel (I) können den Kunststoffen noch weitere Stabilisatoren zugesetzt werden. Derartige weitere Verbindungen sind z.B. Antioxydantien auf der Basis sterisch gehinderter Phenole, oder Schwefel oder Phosphor enthaltende Costabilisatoren oder eine Mischung von geeigneten sterisch gehinderten Phenolen und Schwefel und/oder Phosphor enthaltenden Verbindungen. Solche Verbindungen sind z.B. Benzofuranon(2)- und/oder Indolinon(2)verbindungen, sterisch gehinderte Phenole wie
β-(4-Hydroxy-3,5-di-t-butylphenyl)-propionsäurestearylester, Tetrakis-[methylen-3(3',5'-di-t-butyl-4-hydroxyphenyl-)propionat]-methan,
1,3,3-Tris-(2-methyl-4-hydroxy-5-butylphenyl)-butan,
1,3,5 Tris-(4-t-butyl-3-hydroxy-2,6-dimethylbenzyl)-1,3,5-triazin-2,4,6-(1H, 3H, 5H)-trion,
Bis-(4-t-butyl-3-hydroxy-2,6-dimethylbenzyl)-dithiolterephthalat,
Tris-(3,5-t-butyl-4-hydroxybenzyl)-isocyanurat,
Triester der β-(4-hydroxy-3,5-di-t-butylphenyl)propionsäure mit
1,3,4-Tris-(2-hydroxyethyl)-5-triazin 2,4,6-(1H, 3H, 5H)trion,
Bis-[3,3-bis-(4'-hydroxy-3-t-butylphenyl)-butansäure]-glycolester,
2,3,5-Trimethy-2,4,6-tris-(3,5-di-t-butyl-4-hydroxybenzyl-)benzol,
2,2'-Methylen-bis-(4-methyl-6-t-butylphenyl)-terephthalat,
4,4-Methylen-bis-(2,6-di-t-butylphenol),
4,4'-Butyliden-bis-(-t-butyl-meta-kresol),
4,4-Thio-bis-(2-t-butyl-5-methyl-phenol),
2,2'-Methylen-bis-(4-methyl-6-t-butyl-phenol).
Auch antioxidativ wirkende Co-Stabilisatoren können zugegeben werden, wie z.B. schwefelhaltige Verbindungen, z.B.
Distearylthio-dipropionat,
Dilaurylthiodipropionat,
Tetrakis-(methylen-3-hexyl-thiopropionat)-methan,
Tetrakis-(methylen-3-dodecyl-thiopropionat)-methan
und Dioctadecyldisulfid oder phosphorhaltige Verbindungen wie z.B.
Trinonylphenylphosphit;
4,9-Distearyl-3,5,8,10-tetraoxadiphosphaspiroundecan,
Tris-(2,4-t-butylphenyl)-phosphit oder
Tetrakis-(2,4 di-t-butylphenyl)-4,4'-butylphenyl)-4,4'-biphenylen-diphosphonit.

Die Verbindungen der Formel I und deren oben erwähnte Mischungen kann man auch in Gegenwart weiterer Additive verwenden. Solche sind an sich bekannt und gehören z.B. zur Gruppe der Aminoacrylverbindungen, der UV-Absorber und Lichtschutzmittel wie die 2-(2'-Hydroxyphenyl)-benztriazole, 2-Hydroxybenzophenone, 1,3-Bis(2'-hydroxybenzoyl)-benzole, Salicylate, Zimtsäureester, Ester von gegebenenfalls substituierten Benzoesäuren. sterisch gehinderte Amine, Oxalsäurediamide.

Die Anwendungsmenge der erfindungsgemäß hergestellten Verbindungen der Formel I beträgt 0,1-5 Gew.-% bei Kunststoffen, 20 bis 80 Gew.-% bei Stabilisatorkonzentraten und 0,02-5 Gew.-% bei Lacken.

*Vergleichsbeispiel A*

(Beispiel 1 der DE-OS 3 149 453, aber ohne Reinigung)

91,1 g (0,25 Mol) 2,2,4,4-Tetramethyl-7-oxa-3,20-diazadispiro[5,1,11,2]-heneicosan-21-on und 0,3 g (0,01 Mol) Natrium wurden in 91,1 g Dimethylsulfoxid 2 h auf 120°C erhitzt. Dann wurden innerhalb von 10 min 89,5 g (1 Mol) Acrylsäuremethylester zugetropft und 5 h bei 110°C gerührt. Das Reaktionsgemisch wurde auf Eis/Wasser gegossen und mit Ether extrahiert. Die organische Phase wurde über Magnesiumsulfat getrocknet, abfiltriert und das Lösemittel abdestilliert. Als Rückstand verblieben 100 g eines beigen Feststoffs mit einem Schmelzpunkt von 103°C.

*Vergleichsbeispiel B*

Analog Vergleichsbeispiel A, jedoch mit nur 25,8 g (0,3 Mol) Acrylsäuremethylester. Dieser Versuch ergab 65,5 g hellen Feststoff mit einem Schmelzpunkt von 89°C.

*Vergleichsbeispiel C*

91,1 g (0,25 Mol) 2,2,4,4-Tetramethyl-7-oxa-3,20-diaza-dispiro[5,1,11,2]-heneicosan-21-on und 0,5 g Natrium wurden in 100 g Toluol auf 80°C erhitzt. Innerhalb von 30 min wurden 21,6 g (0,25 Mol) Acrylsäuremethylester zugetropft. Bei 80°C wurde 6 h weitergerührt. Anschließend wurde dreimal mit je 100 g Wasser ausgeschüttelt und aus der organischen Phase das Toluol abdestilliert. Aus dem Produkt wurden durch Umlösen noch 4 g 2,2,4,4-Tetramethyl-7-oxa-3,20-diaza-dispiro[5,1,11,2]-heneicosan-21-on isoliert. Es blieben 89 g heller Feststoff mit einem Schmelzpunkt von 97°C.

*Beispiel 1*

Die Durchführung erfolgte gemäß Vergleichsbeispiel C, jedoch wurden zusätzlich 5 g Triethylbenzylammoniumchlorid zugesetzt. Hier war kein Rückstand von 2,2,4,4-Tetramethyl-7-oxa-3,20-diaza-dispiro-[5,1,11,2]-heneicosan-21-on isolierbar. Man

erhielt als Endprodukt 104 g eines weißen Feststoffs mit einem Schmelzpunkt von 104°C.

*Beispiel 2*

Durchführung wie bei Vergleichsbeispiel C, jedoch mit folgenden Mengenänderungen: 25,8 g (0,3 Mol) Acrylsäuremethylester und 3 g Triethylbenzylammoniumchlorid. Hier konnte kein Rückstand an 2,2,4,4-Tetramethyl-7-oxa-3,20-diazadispiro-[5,1,11,2]heneicosan-21-on isoliert werden. Als Endprodukt wurden 109 g eines weißen Feststoffs mit einem Schmelzpunkt von 114°C erhalten.

*Beispiel 3 bis 5*

Durchführung wie Beispiel 2, jedoch mit folgenden Phasentransferkatalysatoren:

| Beispiel | Phasentransferkatalysator | Endprodukt [g] | Schmelzpunkt [°C] |
|---|---|---|---|
| 3 | 5 g Triethylbenzylammoniumbromid | 96 | 112 |
| 4 | 5 g Triethylbenzylammoniumchlorid | 104 | 108 |
| 5 | 5 g Pentaethylenglykoldimethylether | 112 | 100 |

*Beispiel 6 bis 8 und Vergleichsbeispiel D*

Die Durchführung erfolgte wie in Vergleichsbeispiel C, jedoch wurden 76,5 g (0,3 Mol) Laurylacrylat (technisches Gemisch von ca. 53-55% $C_{12}$-Ester und ca. 42-43% $C_{14}$-Ester) sowie folgende Mengen Triethylbenzylammoniumchlorid eingesetzt:

| Beispiel | Triethylbenzylammoniumchlorid in [g] | Endprodukt in [g] | Restgehalt an 2,2,4,4-Tetramethyl-7-oxa-3,20-diaza-dispiro[5,1,11,2]heneicosan-21-on in Gew.-% |
|---|---|---|---|
| Vergleich D | 0 | 151 | 3,8 |
| 6 | 1 | 158 | 2,7 |
| 7 | 3 | 160 | 0,7 |
| 8 | 5 | 146 | 2,3 |

*Beispiel 9 und 10:*

Die Durchführung erfolgte wie bei Beispiel 6 bis 8 und Vergleichsbeispiel D, jedoch mit Phosphoniumsalzen anstatt von Ammoniumsalzen als Phasentransferkatalysatoren.

| Beispiel | Phasentransferkatalysator | Endprodukt in [g] | Restgehalt an 2,2,4,4-tetramethyl-7-oxa-3,20-diaza-dispiro-[5,1,11,2]-heneicosan-21-on in Gew.-% |
|---|---|---|---|
| 9 | 3,0 g Tributylhexadecylphosphoniumbromid | 160 | 2,6 |
| 10 | 3,8 g Tetrabutylphosphoniumbromid | 163 | 0,8 |

**Patentansprüche**

1. Verfahren zur Herstellung von 1-Oxa-3,8-diaza-4-oxo-spiro-[4,5]decan-Verbindungen der Formel I

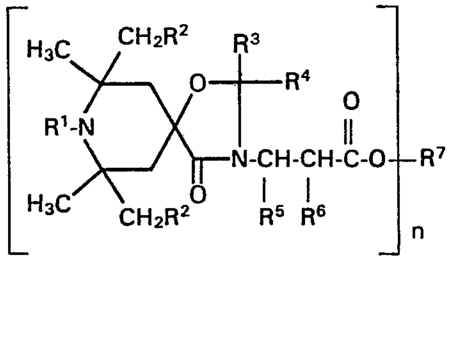

(I)

worin

n eine ganze Zahl von 1 bis 4 ist,

$R^1$ Wasserstoff, $C_1$-$C_4$-Alkyl, Benzyl, Allyl, $C_2$-$C_{30}$-Alkanoyl, $C_3$-$C_{20}$-Alkenoyl, $C_7$-$C_{11}$-Aroyl, $C_8$-$C_{14}$-Arylalkanoyl oder $C_8$-$C_{20}$-Alkylaryl ist.

$R^2$ Wasserstoff oder $C_1$-$C_4$-Alkyl bedeutet,

$R^3$ Wasserstoff, $C_1$-$C_{18}$-Alkyl, $C_5$-$C_{12}$-Cycloalkyl, eine Phenyl- oder Naphthylgruppe, die durch Chlor oder $C_1$-$C_4$-Alkyl substituiert sein kann, oder eine gegebenenfalls durch $C_1$-$C_4$-Alkyl substituierte $C_7$-$C_{12}$-Phenylalkylengruppe ist,

$R_4$ Wasserstoff, $C_1$-$C_4$-Alkyl, $C_5$-$C_{12}$-Cycloalkyl, durch -COOH, Carb-$C_1$-$C_4$-alkoxi oder Carbamoyl substituiertes $C_1$-$C_3$-Alkenyl, eine Phenyl-, Naphthyl- oder Pyridylgruppe, die durch $C_1$-$C_4$-Alkoxi oder $C_1$-$C_4$-Alkyl substituiert sein kann, oder eine

$C_7$-$C_{12}$-Phenylalkylgruppe, die durch $C_1$-$C_4$-Alkyl substituiert sein kann, bedeutet, oder

$R^3$ und $R^4$ zusammen mit dem sie bindenden Kohlenstoffatom eine Cycloalkylgruppe, die durch eine bis vier $C_1$-$C_4$-Alkylgruppen substituiert sein kann, oder einen Rest der Formel II

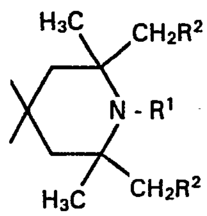

(II)

worin $R^1$ und $R^2$ die obengenannte Bedeutung haben, bilden

$R^5$ Wasserstoff, Methyl, Phenyl oder Carb-$C_1$-$C_{21}$-Alkoxi ist,

$R^6$ Wasserstoff oder Methyl bedeutet,

$R^7$ bei n = 1

Wasserstoff, $C_1$-$C_{21}$-Alkyl, $C_2$-$C_{22}$-Alkenyl, $C_7$-$C_{18}$-Phenylalkyl, $C_5$-$C_{12}$-Cycloalkyl, Phenyl, Naphthyl, $C_7$-$C_{18}$-Alkylphenyl, ein Rest der Formel

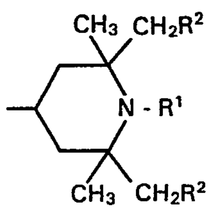

worin $R^1$ und $R^2$ die oben angegebene Bedeutung haben, $C_2$-$C_{20}$-Alkyl, welches unterbrochen ist durch -O- oder $-\underset{\substack{|\\R^8}}{N}-$ und/oder substituiert durch einen Rest der Formel III

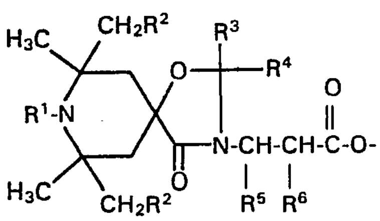

(III),

oder durch $C_1$-$C_{21}$-Alkylcarbonyl, wobei $R^1$, $R^2$, $R^3$, $R^4$, $R^5$ und $R^6$ die obige Bedeutung haben und $R^8$ Wasserstoff oder $C_1$-$C_{10}$-Alkyl ist, bedeutet,

$R^7$ bei n = 2

geradkettiges oder verzweigtes $C_1$-$C_{30}$-Alkylen, $C_2$-$C_{30}$-Alkenylen, Phenyldialkylen, wobei diese Reste durch -O- oder $-\underset{\substack{|\\R^8}}{N}-$, worin $R^8$ die obige Bedeutung hat, unterbrochen sein können, bedeutet,

$R^7$ bei n = 3 oder 4

einen Rest der Formeln IV, V, VI oder VII

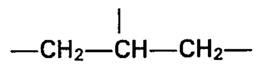

(IV),

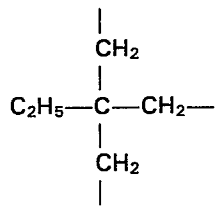

(V),

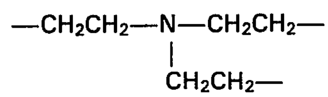

(VI),

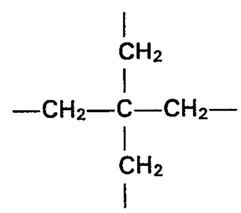

(VII),

bedeutet,

durch Umsetzung von Verbindungen der Formel VIII

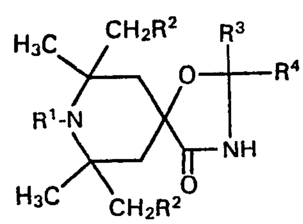

(VIII),

mit Verbindungen der Formel IX

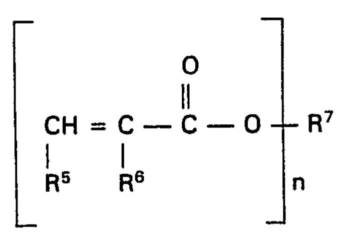

(IX),

Wobei $R^1$, $R^2$, $R^3$, $R^4$, $R^5$, $R^6$ und $R^7$ die oben genannte Bedeutung haben, in einer Menge von 1/n bis 10/n, wobei n eine ganze Zahlk von 1 bis 4 ist, bezogen auf 1 mol der Verbindung der Formel VIII, in einem inerten Lösemittel bei einer Temperatur von 30 bis 150°C in Gegenwart von 1 bis 30 Mol-%, bezogen auf eine Verbindung der Formel VIII, eines basischen Katalysators, dadurch gekennzeichnet, daß die Umsetzung in Gegenwart von 0,05 bis 20 Mol-%, bezogen auf die Verbindung VIII, eines Phasentransferkatalysators in einem bei Raumtemperatur flüssigen aromatischen Kohlenwasserstoff durchgeführt wird.

2. Verfahren nach Anspruch 1, dadurch gekennzeichnet, daß der als Reaktionsmedium dienende aromatische Kohlenwasserstoff Toluol oder Xylol ist.

3. Verfahren nach Anspruch 1, dadurch gekennzeichnet, daß man als Phasentransferkatalysator ein substituiertes Phosphoniumsalz oder Ammoniumsalz oder einen Polyethylenglykoldialkylether verwendet.

4. Verfahren nach Anspruch 3, dadurch gekennzeichnet, daß man als Phasentransferkatalysator ein Tetraalkyl- oder Trialkylbenzylammoniumchlorid verwendet.

5. Verfahren nach Anspruch 3, dadurch gekennzeichnet, daß man als Phasentransferkatalysator ein Tetraalkylphosphoniumbromid verwendet.

## Claims

1. A process for the preparation of a 1-oxa-3,8-diaza-4-oxospiro[4.5]decane compound of the formula I

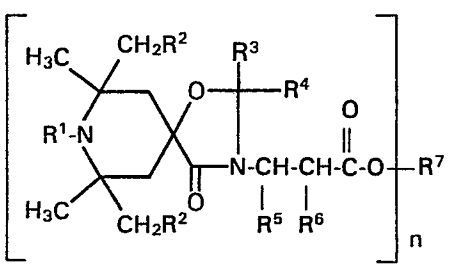

(I)

wherein

n is an integer from 1 to 4,

$R^1$ is hydrogen, $C_1$-$C_4$ alkyl, benzyl. allyl, $C_2$-$C_{30}$ alkanoyl, $C_3$-$C_{20}$ alkenoyl, $C_7$-$C_{11}$ aroyl, $C_8$-$C_{14}$ arylalkanoyl or $C_8$-$C_{20}$ alkylaryl,

$R^2$ denotes hydrogen or $C_1$-$C_4$ alkyl,

$R^3$ is hydrogen, $C_1$-$C_{18}$ alkyl, $C_5$-$C_{12}$ cycloalkyl, a phenyl or naphthyl group which may be substituted by chlorine or $C_1$-$C_4$ alkyl, or a $C_7$-$C_{12}$ phenylalkylene group optionally substituted by $C_1$-$C_4$ alkyl,

$R^4$ denotes hydrogen, $C_1$-$C_4$ alkyl, $C_5$-$C_{12}$ cycloalkyl, $C_1$-$C_3$ alkenyl substituted by -COOH, carb($C_1$-$C_4$ alkoxy) or carbamoyl, a phenyl, naphthyl or pyridyl group which may be substituted by $C_1$-$C_4$ alkoxy or $C_1$-$C_4$ alkyl, or a $C_7$-$C_{12}$ phenylalkyl group which may be substituted by $C_1$-$C_4$ alkyl, or

$R^3$ and $R^4$ together with the carbon atom which binds them may form a cycloalkyl group which may be substituted by one to four $C_1$-$C_4$ alkyl groups, or a radical of formula II

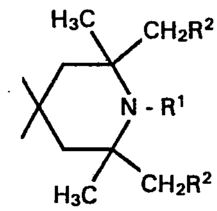

(II)

wherein $R^1$ and $R^2$ have the abovementioned meaning,

$R^5$ is hydrogen, methyl, phenyl or carb($C_1$-$C_{21}$ alkoxy),

$R^6$ denotes hydrogen or methyl,

$R^7$ denotes, for n = 1,
hydrogen, $C_1$-$C_{21}$ alkyl, $C_2$-$C_{22}$ alkenyl, $C_7$-$C_{18}$ phenylalkyl, $C_5$-$C_{12}$ cycloalkyl, phenyl, naphthyl, $C_7$-$C_{18}$ alkylphenyl, a radical of the formula

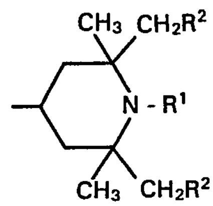

in which $R^1$ and $R^2$ have the meaning specific above, $C_2$-$C_{20}$ alkyl which is interrupted by -O- or -N- and/or
|
$R^8$
substituted by a radical of the formula III

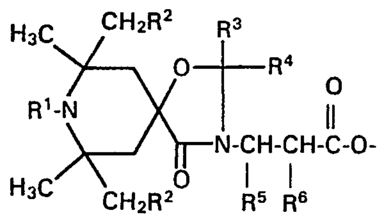

(III),

or by $C_1$-$C_{21}$ alkylcarbonyl, $R^1$, $R^2$, $R^3$, $R^4$, $R^5$ and $R^6$ having the meaning specific above and $R^8$ being hydrogen or $C_1$-$C_{10}$ alkyl,

$R^7$ denotes, for n = 2,
straight-chain or branched $C_1$-$C_{30}$ alkylene, $C_2$-$C_{30}$ alkenylene, phenyldialkylene, it being possible for these radicals to be interrupted by -O- or -N-,
|
$R^8$
wherein $R^8$ has the meaning specific above,

$R^7$ denotes, for n = 3 or 4,
a radical of the formulae IV, V, VI or VII

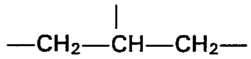
(IV),

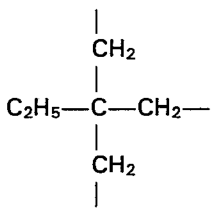
(V),

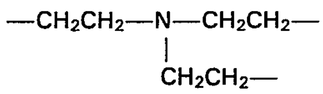
(VI),

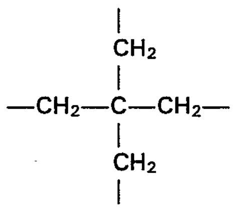
(VII),

by reaction of a compound of the formula VIII

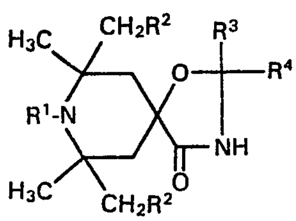
(VIII),

with a compound of the formula IX

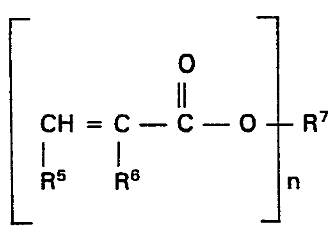
(IX),

where $R^1$, $R^2$, $R^3$, $R^4$, $R^5$, $R^6$ and $R^7$ have the above-mentioned meaning, in an amount of 1/n to 10/n, n being an integer from 1 to 4, based on 1 mol of the compound of the formula VIII, in an inert solvent at a temperature of 30 to 150°C in the presence of 1 to 30 mol %, based on a compound of the formula VIII, of a basic catalyst, wherein the reaction is performed in the presence of 0.05 to 20 mol %, referred to the compound VIII, of a phase transfer catalyst in an aromatic hydrocarbon which is liquid at room temperature.

2. The process as claimed in claim 1, wherein the aromatic hydrocarbon serving as reaction medium is toluene or xylene.

3. The process as claimed in claim 1, wherein a substituted phosphonium salt or ammonium salt or a polyethylene glycol dialkyl ether is used as phase transfer catalyst.

4. The process as claimed in claim 3, wherein a tetraalkyl or trialkylbenzylammonium chloride is used as phase transfer catalyst.

5. The process as claimed in claim 3, wherein a tetraalkylphosphonium bromide is used as phase transfer catalyst.

**Revendications**

1. Procédé pour préparer des oxa-1 diaza-3,8 oxo-4 spiro[4.5]décanes répondant à la formule I:

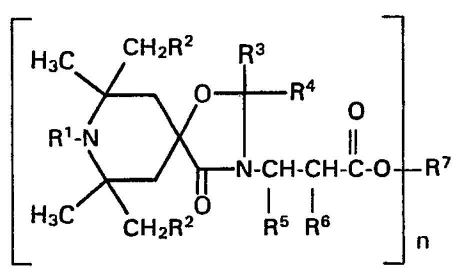
(I)

dans laquelle

n désigne un nombre entier de 1 à 4,

$R^1$ représente l'hydrogène, un alkyle en $C_1$-$C_4$, un benzyle, un allyle, un alcanoyle en $C_2$-$C_{30}$, un alcénoyle en $C_3$-$C_{20}$, un aroyle en $C_7$-$C_{11}$, un arylalcanoyle en $C_8$-$C_{14}$ ou un alkylaryle en $C_8$-$C_{20}$,

$R^2$ représente l'hydrogène ou un alkyle en $C_1$-$C_4$,

$R^3$ représente l'hydrogène, un alkyle en $C_1$-$C_{18}$, un cycloalkyle en $C_5$-$C_{12}$, un radical phényle ou naphthyle éventuellement porteur d'un atome de chlore ou d'un alkyle en $C_1$-$C_4$, ou un radical phénylalkyle en $C_7$-$C_{12}$ éventuellement porteur d'un alkyle en $C_1$-$C_4$,

$R^4$ représente l'hydrogène, un alkyle en $C_1$-$C_4$, un cycloalkyle en $C_5$-$C_{12}$, un alcényle en $C_1$-$C_3$ porteur d'un radical -COOH, d'un alcoxycarbonyle à alcoxy en $C_1$-$C_4$ ou d'un carbamoyle, un radical phényle, naphthyle ou pyridyle éventuellement porteur d'un alcoxy en $C_1$-$C_4$ ou d'un alkyle en $C_1$-$C_4$, ou un phénylalkyle en $C_7$-$C_{12}$ qui peut porter un alkyle en $C_1$-$C_4$, ou

$R^3$ et $R^4$ forment ensemble, et avec l'atome de carbone auxquels ils sont liés, un radical cycloalkyle éventuellement porteur d'un à quatre alkyles en $C_1$-$C_4$, ou un radical de formule II:

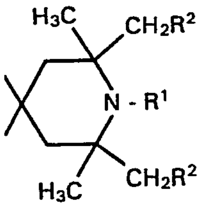 (II),

dans lequel $R^1$ et $R^2$ ont les significations qui leur ont été données ci-dessus,

$R^5$ représente l'hydrogène, un méthyle, un phényle ou un alcoxycarbonyle à alcoxy en $C_1$-$C_{21}$,

$R^6$ représente l'hydrogène ou un méthyle,

$R^7$ représente, dans le cas où n est égal à 1, l'hydrogène, un alkyle en $C_1$-$C_{21}$, un alcényle en $C_2$-$C_{22}$, un phénylalkyle en $C_7$-$C_{18}$, un cycloalkyle en $C_3$-$C_{12}$, un phényle, un naphthyle, un alkylphényle en $C_7$-$C_{18}$, un radical de formule:

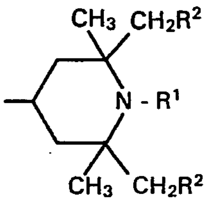

dans lequel $R^1$ et $R^2$ ont les significations qui leur ont été données ci-dessus, un alkyle en $C_2$-$C_{20}$ qui est interrompu par -O- ou -N- (portant $R^8$) et/ou qui porte un radical de formule III:

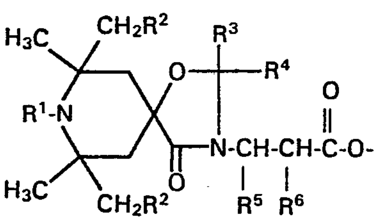 (III),

dans lequel $R^1$, $R^2$, $R^3$, $R^4$, $R^5$ et $R^6$ ont les significations précédemment données et $R^8$ représente l'hydrogène ou un alkyle en $C_1$-$C_{10}$, ou porte un $C_1$-$C_{21}$-alkylcarboxyle,

$R^7$ représente, dans le cas où n est égal à 2, un alkylène en $C_1$-$C_{30}$ linéaire ou ramifié, un alcénylène en $C_2$-$C_{30}$ ou un phényl-dialkylène, ces radicaux pouvant être interrompus par -O- ou -N-, le symbole (portant $R^8$)

$R^8$ ayant la signification précédemment donnée,

$R^7$ représente, dans le cas où n est égal à 3 ou à 4, un radical répondant à l'une des formules IV, V, VI et VII:

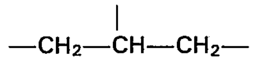 (IV),

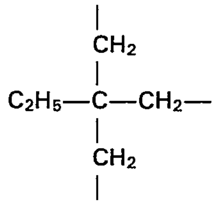 (V),

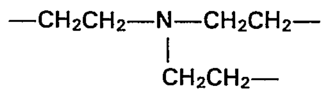 (VI),

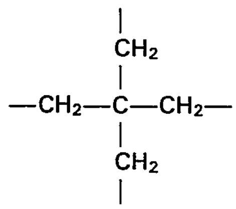 (VII),

par réaction de composé de formule VIII:

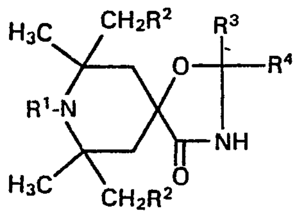 (VIII),

avec des composés de formule IX:

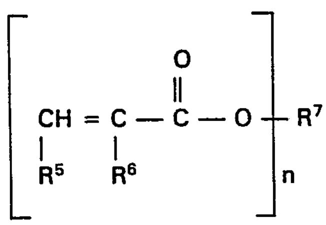 (IX),

formules dans lesquelles $R^1$, $R^2$, $R^3$, $R^4$, $R^5$, $R^6$ et $R^7$ ont les significations qui leur ont été données ci-dessus, en une quantité comprise entre 1/n et 10/n, où n désigne un nombre entier de 1 à 4, pour 1 mol du composé de formule VIII, dans un solvant inerte, à une température de 30 à 150°C, en présence de 1 à 30% en moles, par rapport à un composé de formule VIII, d'un catalyseur basique, procédé caractérisé en ce qu'on effectue la réaction en présence de 0,05 à 20% en moles, par rapport au composé de formule VIII, d'un catalyseur de transfert de phase, dans un hydrocarbure aromatique liquide à la température ambiante.

2. Procédé selon la revendication 1, caractérisé en ce que l'hydrocarbure aromatique qui sert de milieu réactionnel est le toluène ou un xylène.

3. Procédé selon la revendication 1 caractérisé en ce qu'on utilise, comme catalyseur de transfert de

phase, un sel de phosphonium substitué, un sel d'ammonium substitué ou un éther dialkylique d'un polyéthylène-glycol.

4. Procédé selon la revendication 3 caractérisé en ce qu'on utilise, comme catalyseur de transfert de phase, un chlorure de tétra-alkyl-ammonium ou un chlorure de trialkyl-benzyl-ammonium.

5. Procédé selon la revendication 3 caractérisé en ce qu'on utilise, comme catalyseur de transfert de phase, un bromure de tétraalkyl-phosphonium.